# EUROPEAN PATENT SPECIFICATION

(11) **EP 1 485 157 B1**
(45) Date of publication and mention of the grant of the patent: **28.03.2007**
(21) Application number: 03744437.9
(22) Date of filing: 12.03.2003
(51) Int. Cl.: A61M 16/22

(54) **APPARATUS FOR HYPOXIC TRAINING AND THERAPY**
EINRICHTUNG FÜR HYPOXISCHES TRAINING UND THERAPIE
APPAREIL D'ENTRAINEMENT ET DE TRAITEMENT HYPOXIQUE

(30) Priority: 12.03.2002 GB 0205759
(43) Date of publication of application: 15.12.2004
(73) Proprietor: SOUTH BANK UNIVERSITY ENTERPRISES LTD., London SE1 0AA (GB)
(72) Inventor: SUMNERS, David Paul, Middlesbrough, Cleveland TS6 0HX (GB)
(74) Representative: Lucas, Brian Ronald
(86) International application number: PCT/GB2003/001041
(87) International publication number: WO 2003/077980

(56) References cited:
- RU-C1- 2 040 279
- US-A- 5 228 435
- US-A- 5 850 833
- US-A- 5 983 896

## Description

This invention relates to equipment for improving the breathing of people such as athletes, singers, people with breathing difficulties and anyone who wants to improve the efficiency of their breathing and endurance.

Athletes, particularly those who take part in middle and long distance events, often train at high altitudes as such high altitude training is known to improve their performance. This improvement is thought to be due to the lower oxygen levels at high altitudes resulting in the body having to become more efficient in its operations.

An acclimated athlete can run at high altitudes because the body can adapt to hypocapnia. This adaptation permits greatly increased ventilation which supplies enough O₂ not only to prevent hypoxia at rest but also provides enough ventilation for strenuous running. This adaptation brings about improved performance at lower altitudes.

However, this adaptive process does not always go smoothly, and acute mountain sickness is a common occurrence. At high altitudes, the alternating stimulation and inhibition of the respiratory centre, first by hypoxia and then by hypocapnia, leads to Cheyne-Stokes respiration, which can become quite pronounced during sleep. In the apneic phase, severe hypoxia may potentially cause the subject to slip from sleep into coma, and sometimes from coma into death.

A voluntary increase in the rate and depth of breathing causes CO₂ to be exhaled at a faster rate than its rate of production by the body's metabolism and results in a drop in the amount of CO₂ in the blood, i.e., results in hypocapnia. If vigorous, rapid breathing is continued for more than a few minutes, increasingly severe hypocapnia will cause cerebral vasoconstriction and unpleasant nervous system symptoms.

An increased rate and depth of breathing, or hyperpnoea, without an appropriate increase in CO₂ production from metabolism, can be voluntary or caused by a hyperventilation syndrome, anoxic hypoxia, or mechanical ventilation. In all cases, the resultant hypocapnia causes increasingly grave symptoms and is the limiting factor in the amount of excess ventilation that can be achieved. In a number of situations-a good example is the anoxic hypoxia that can occur in high altitude flying-a large increase in ventilation is desirable, and CO₂ enriched air makes this possible.

Various attempts to utilize exhaled air, which is high in CO₂, have been made as a substitute for providing prepared custom mixes of CO₂ and air. In fact, generations of emergency room physicians have had patients breathe into simple Kraft paper bags to treat hyperventilation that can result from anxiety, fear, or trauma. The paper bag enables a hyperventilating patient to conserve and rebreathe exhaled air.

Variations on the use of paper bags are described in US Patents 3,455,294; 4,508,116 and 4,628,926 and in RU-C-2040297. Long tubes have been substituted for paper bags and these tubes essentially mimic the effect of paper bags. These devices cannot be used to adjust the air breathed to reproduce the air at a selected altitude.

US Pat. No. 5,850,833 shows an apparatus for hypoxic training and therapy disclosing all features of the preamble of claim 1.

U.S. Pat. No. 4,275,722 discloses a respiratory exerciser and rebreathing device which, through a system of valves, provides for an inhalation chamber and an exhalation chamber, with a sliding mechanism to vary the amount of air rebreathed from the exhalation chamber. This device has a complex network of chambers, valves and mechanisms, all designed to route exhaled air through an exhalation chamber and through an inhalation chamber that removes moisture from the exhaled air before inhaling. The exhalation chamber is widely open to ambient air so that fresh air is available at the bottom.

US Patent 5983896 discloses apparatus for controlling the carbon dioxide levels in rebreathed air, but does not disclose the reduction of oxygen levels for reproducing the atmosphere at altitude for training purposes. The apparatus comprises a conduit playing role of a dead space for collecting expired CO₂ to be rebreathed, the conduit being formed of an adjustable-length tubing, so that the dead space can be adjusted.

These devices all are designed to combat the effects of breathing problems at high altitude and to overcome physiological difficulties and cannot be used to reproduce the effect of high altitude training.

Efforts to reproduce the effect of high altitude training at lower altitudes, in order to avoid the expense of travel to, and living in places of high altitudes by training in rooms or chambers with reduced air pressure are expensive to set up and operate and inconvenient to use. Restricting the airflow to an athlete whilst he or she is training is not effective as the volume of air taken with each breath is reduced, which can cause adverse effects on the athlete. Existing equipment which involves the use of rebreathing air so that the air has a lower oxygen content is not practical as this can lead to excessive carbon dioxide build up as detailed above.

We have now devised a simple effective device for at least partially reproducing the effect of high altitude training which dies not suffer from these disadvantages.

According to the invention there is provided breathing equipment which comprises a mouthpiece through which a user can breath, which mouthpiece is connected to the inlet of a chamber containing a carbon dioxide absorber, the outlet of the chamber being connected to a conduit which is open to the atmosphere in which, in use, the air in said conduit comprises a mixture of air which has been breathed out by the user and air from the atmosphere, which mixture is breathed in by the user.

The carbon dioxide absorber can be any of the conventionally used carbon dioxide absorbers such as caustic soda pellets, soda lime etc. Preferably the carbon dioxide absorber changes colour as it absorbs carbon dioxide and so it can be seen when it is used up.

The conduit can be a flexible tube and the length of the conduit depends on the amount of air from the atmosphere it is desired to add to the air to be re-breathed, with the longer the conduit the less fresh air form the atmosphere is added on each breath. For tubes of diameter 1.5 cm to 4 cm tubes of lengths of 50cm to 1.5 metres can be used.

The air from the atmosphere enters the conduit by diffusion and by the reduction in pressure caused by each in-breath.

In use, the mouthpiece is attached to the user's face e.g. by being tied on means of straps or an elasticated bands etc. As there is a reduction in oxygen input in use there is preferably an automatic release mechanism so that, in the event of discomfort, air can enter directly into mouthpiece.

In use the air is breathed out by the user and passes through the carbon dioxide absorber chamber where excess carbon dioxide is absorbed, and then into the conduit, where it mixes with air from the atmosphere. This air is breathed in through the carbon dioxide absorber chamber and the air breathed will consist of air with an oxygen and carbon dioxide content similar to that found at high altitude. By adjustment of the length of the conduit and the carbon dioxide absorber chamber contents, the conditions at a selected altitude can be reproduced. This enables a graduated acclimatisation to high altitude conditions to be achieved and is equivalent to high altitude training.

This patent application excludes the use of the method of the invention for therapeutic purposes and the use of the method of the invention for treatment of the human body by therapy; such uses and applications are hereby disclaimed.

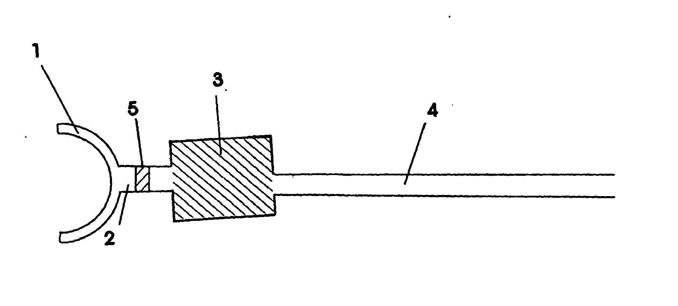

The invention is illustrated in the accompanying drawing in which there is a mouthpiece (1) connected by tube (2) to a chamber (3) containing soda lime. At the exit of chamber (3) is a flexible tube (4).

In use, a user straps the mouthpiece over his face so that the user breathes in and out through the mouthpiece (1). When a user breathes out the air breathed out by the user passes through the carbon dioxide absorber chamber (3), where excess carbon dioxide is absorbed, and then into the tube (4), where it mixes with air from the atmosphere. This air is then breathed in through the carbon dioxide absorber chamber (3) and the air breathed in will consist of air with an oxygen and carbon dioxide content similar to that found at high altitude. By adjustment of the length of the conduit (4) and the carbon dioxide absorber chamber (3) contents, the conditions at a selected altitude can be reproduced. This enables a graduated acclimatisation to high altitude conditions to be achieved and is equivalent to high altitude training.

There is release valve (5) which can be actuated to open an air inlet directly into the mouthpiece in case of discomfort or danger; such a valve can be actuated automatically under specified conditions.

## Claims

1. Breathing equipment for training the breathing of a user by varying the composition of the air breathed in which comprises (i) a mouthpiece (1) through which a user can breathe in and out and (ii) a chamber (3) containing a carbon dioxide absorber, in which the mouthpiece is connected to the inlet of the chamber and the outlet of the chamber is connected to a conduit (4) which is open to the atmosphere and in use, the air in said conduit comprises a mixture of air which has been breathed out by the user and air from the atmosphere, which mixture is breathed in by the user through the chamber **characterised in that** the conduit (4) is configured such that its length is adjustable, and by varying the length of the conduit, the amount of the air which has been breathed out by the user and which is mixed with the atmospheric air can be varied so that the proportion of oxygen and carbon dioxide in the air breathed in can be varied to progressively train the breathing of a user.

2. Breathing equipment as claimed in claim 1 **characterised in that** the carbon dioxide absorber comprises caustic soda pellets or soda lime modified so that the absorber changes colour as it absorbs carbon dioxide.

3. Breathing equipment as claimed in claim 1 or 2 **characterised in that** there are attachment means to attach the mouthpiece to the face of a user.

4. Breathing equipment as claimed in claim 3 **characterised in that** the attachment means are straps, an elasticated band or the like.

5. Breathing equipment as claimed in any one of claims 1 to 4 **characterised in that** the conduit comprises a tube (4) of diameter of 1.5 cm to 4 cm and a length of between 50cm to 1.5 metres.

6. Breathing equipment as claimed in any one of claims 1 to 5 **characterised in that** there is a release mechanism (5) which, when actuated, enables air to enter directly into mouthpiece without passing through the carbon dioxide absorber.

7. A method for breath training other than for therapeutic purposes **characterised in that** air breathed out by the user passes through a carbon dioxide absorber in a chamber where excess carbon dioxide is absorbed, and then into a conduit open to the atmosphere, in which conduit the air breathed out is mixed with air from the atmosphere, this air mixture is then breathed in through the carbon dioxide absorber and, by varying the length of the conduit, the oxygen content of the air breathed in is varied.

8. A method as claimed in claim 7 in which the length of the conduit and the content of the carbon dioxide absorber in the chamber are adjusted so that the air breathed in consists of air with an oxygen and carbon dioxide content similar to that found at a high altitude.

9. A method as claimed in claim 7 **characterised in that** the conduit comprises a tube of diameter of 1.5 cm to 4 cm and a length of 50cm to 1.5 metres.

## Patentansprüche

1. Atmungsgerät zum Trainieren der Atmung eines Benutzers durch Verändern der Zusammensetzung der eingeatmeten Luft, die umfasst (i) ein Mundstück (1), durch das ein Benutzer ein- und ausatmen kann, und (ii) eine Kammer (3), die einen Kohlendioxidabsorber enthält, wobei das Mundstück mit dem Einlass der Kammer verbunden ist und der Auslass der Kammer mit einer Leitung (4) verbunden ist, die zur Atmosphäre mündet, im Gebrauch die Luft in der Leitung ein Gemisch aus Luft, die von dem Benutzer ausgeatmet worden ist, und Atmosphärenluft enthält und das Gemisch von dem Benutzer durch die Kammer eingeatmet wird, **dadurch gekennzeichnet, dass** die Leitung (4) so konfiguriert ist, dass ihre Länge einstellbar ist, und durch Ändern der Länge der Leitung die Menge der Luft, die von dem Benutzer ausgeatmet worden ist und mit der Atmosphärenluft vermischt wird, geändert werden kann, so dass der Anteil von Sauerstoff und von Kohlendioxid in der eingeatmeten Luft so geändert werden kann, dass die Atmung eines Benutzers progressiv trainiert wird.

2. Atmungsgerät nach Anspruch 1, **dadurch gekennzeichnet, dass** der Kohlendioxidabsorber Ätznatron-Pellets oder modifizierten Natronkalk enthält so dass der Absorber seine Farbe ändert, wenn er Kohlendioxid absorbiert.

3. Atmungsgerät nach Anspruch 1 oder 2, **dadurch gekennzeichnet, dass** Befestigungsmittel vorhanden sind, um das Mundstück am Gesicht eines Benutzers zu befestigen.

4. Atmungsgerät nach Anspruch 3, **dadurch gekennzeichnet, dass** die Befestigungsmittel Riemen, ein elastisches Band oder dergleichen sind.

5. Atmungsgerät nach einem der Ansprüche 1 bis 4, **dadurch gekennzeichnet, dass** die Leitung ein Rohr (4) mit einem Durchmesser im Bereich von 1,5 cm bis 4 cm und mit einer Länge im Bereich von 50 cm bis 1,5 Meter aufweist.

6. Atmungsgerät nach einem der Ansprüche 1 bis 5, **dadurch gekennzeichnet, dass** ein Freigabemechanismus (5) vorhanden ist, der, wenn er betätigt wird, ermöglicht, dass Luft direkt in das Mundstück eintritt, ohne sich durch den Kohlendioxidabsorber zu bewegen.

7. Verfahren zum Atemtraining für andere als therapeutische Zwecke, **dadurch gekennzeichnet, dass** die von dem Benutzer ausgeatmete Luft sich durch einen Kohlendioxidabsorber in einer Kammer bewegt, in der überschüssiges Kohlendioxid absorbiert wird, und sich dann in eine in die Atmosphäre mündende Leitung bewegt, wobei in der Leitung die ausgeatmete Luft mit Atmosphärenluft vermischt wird, wobei dieses Luftgemisch dann über den Kohlendioxidabsorber eingeatmet wird und wobei durch Ändern der Länge der Leitung der Sauerstoffgehalt der eingeatmeten Luft geändert wird.

8. Verfahren nach Anspruch 7, bei dem die Länge der Leitung und der Gehalt des Kohlendioxidabsorbers in der Kammer so eingestellt werden, dass die eingeatmete Luft aus Luft mit einem Sauerstoff- und einem Kohlendioxidgehalt, der ähnlich jenem ist, der auf großer Höhe vorgefunden wird, besteht.

9. Verfahren nach Anspruch 7, **dadurch gekennzeichnet, dass** die Leitung ein Rohr mit einem Durchmesser im Bereich von 1,5 cm bis 4 cm und einer Länge im Bereich von 50 cm bis 1,5 Metern aufweist

## Revendications

1. Equipement respiratoire pour entraîner un utilisateur à la respiration en faisant varier la composition de l'air inspiré, qui comprend (i) un embout buccal (1) par l'intermédiaire duquel un utilisateur peut inspirer et expirer et (ii) une chambre (3) contenant un absorbeur de dioxyde de carbone, dans lequel l'embout buccal est relié à l'entrée de la chambre et la sortie de la chambre est reliée à un conduit (4) qui est ouvert vers l'atmosphère et, en conditions d'utilisation, l'air traversant ledit conduit comprend un mélange d'air qui a été expiré par l'utilisateur et de l'air provenant de l'atmosphère, lequel mélange est inspiré par l'utilisateur à travers la chambre, **caractérisé en ce que** le conduit (4) est configuré de sorte que sa longueur est réglable et **en ce qu'**en faisant varier la longueur du conduit, on peut faire varier la quantité d'air qui a été expirée par l'utilisateur et qui est mélangée à l'air atmosphérique de sorte que l'on peut faire varier la proportion d'oxygène et de dioxyde de carbone dans l'air inspiré pour entraîner progressivement un utilisateur à la respiration.

2. Equipement respiratoire selon la revendication 1, **caractérisé en ce que** l'absorbeur de dioxyde de carbone comprend des granulés de soude caustique ou de la chaux sodée modifiée pour que l'absorbeur change de couleur lorsqu'il absorbe le dioxyde de carbone.

3. Equipement respiratoire selon la revendication 1 ou 2, **caractérisé en ce que** des moyens d'attachement sont prévus pour attacher l'embout buccal au visage d'un utilisateur.

4. Equipement respiratoire selon la revendication 3, **caractérisé en ce que** les moyens d'attache sont des sangles, un élastique ou analogue.

5. Equipement respiratoire selon l'une quelconque des revendications 1 à 4, **caractérisé en ce que** le conduit comprend un tube (4) d'un diamètre de 1,5 cm à 4 cm et d'une longueur comprise entre 50 cm et 1,5 m.

6. Equipement respiratoire selon l'une quelconque des revendications 1 à 5, **caractérisé en ce qu'**un mécanisme de libération (5) qui, lorsqu'il est actionné, permet à l'air de pénétrer directement dans l'embout buccal sans traverser l'absorbeur de dioxyde de carbone.

7. Procédé d'entraînement à la respiration autre qu'à des fins thérapeutiques, **caractérisé en ce que** l'air expiré par l'utilisateur traverse un absorbeur de dioxyde de carbone dans une chambre où le dioxyde de carbone en excès est absorbé, puis dans un conduit ouvert vers l'atmosphère, conduit dans lequel l'air expiré est mélangé à de l'air provenant de l'atmosphère, ce mélange d'air est ensuite inspiré à travers l'absorbeur de dioxyde de carbone et, en faisant varier la longueur du conduit, on fait varier la teneur en oxygène de l'air inspiré.

8. Procédé selon la revendication 7, dans lequel la longueur du conduit et la quantité d'absorbeur de dioxyde de carbone dans la chambre sont ajustés pour que l'air inspiré soit de l'air ayant une teneur en oxygène et en dioxyde de carbone similaire à celle rencontrée à haute altitude.

9. Procédé selon la revendication 8, **caractérisé en ce que** le conduit comprend un tube d'un diamètre de 1,5 cm à 4 cm et une longueur de 50 cm à 1,5 m.
